# EUROPEAN PATENT SPECIFICATION

(11) **EP 4 137 189 B1**
(45) Date of publication and mention of the grant of the patent: **27.11.2024**
(21) Application number: 21817258.3
(22) Date of filing: 26.03.2021
(51) Int. Cl.: A61M 25/00

(54) **CATHETER**
KATHETER
CATHÉTER

(30) Priority: 01.06.2020 JP 2020095666
(43) Date of publication of application: 22.02.2023
(73) Proprietor: TERUMO Kabushiki Kaisha, Tokyo 151-0072 (JP)
(72) Inventor: MOTOSE, Yuji, Ashigarakami-gun Kanagawa 259-0151 (JP); SHIMADA, Daisuke, Fujinomiya-shi Shizuoka 418-0015 (JP)
(74) Representative: Casalonga
(86) International application number: PCT/JP2021/012978
(87) International publication number: WO 2021/246031

(56) References cited:
- WO-A1-2014/103599
- WO-A1-2014/103599
- WO-A1-2016/092208
- JP-A- S6 096 265
- US-A1- 2009 157 007
- US-A1- 2011 139 754
- US-A1- 2011 139 754
- US-A1- 2017 340 860

## Description

### Technical Field

The present invention relates to a catheter. In particular, the present invention relates to a catheter according to the preamble of claim 1, such as it is e.g. known from WO 2016 / 092208 A, US 2009 / 157007 A1, WO 2014 193599 A1 or US 2011 / 139754 A1.

### Background Art

In recent years, treatment of the inside of a lumen such as a blood vessel by using a catheter has been actively performed since surgical invasiveness thereof is very low. The catheter generally includes a shaft including a lumen extending the shaft from a distal end to a proximal end, and a hub disposed at the proximal end of the shaft. The hub is formed with a passage communicating with the lumen in order to connect to a syringe or the like.

As a method for fixing the proximal end of the shaft to the hub, an insert molding method, a bonding method using an adhesive, and the like are known.

### Citation List

### Patent Literature

PTL 1: JP-A-H10-180802
PTL 2: JP-UM-B-S63-17486

### Summary of Invention

### Technical Problem

In an insert molding method described in PTL 1, a shaft is disposed in an injection mold, a part of the shaft is pressed by a fixing pin, and a resin for a hub is injection-molded at a high temperature and a high pressure. Therefore, deformation of the shaft caused by the fixing pin or displacement of the shaft in an axial center direction may occur. The deformation of the shaft and the displacement in the axial center direction may cause a decrease in fixing strength between the shaft and the hub.

In addition, in a bonding method using an adhesive described in PTL 2, if a gap between an outer diameter of a shaft and a lumen of a shaft accommodation unit of a hub is excessively small, the adhesive cannot flow therein, and the gap remains between the hub and the shaft, which may cause a decrease in fixing strength between the shaft and the hub. If the gap between the outer diameter of the shaft and the lumen of the shaft accommodation unit of the hub is excessively large, it is difficult to completely fill the gap between the hub and the shaft with the adhesive, and thus the fixing strength between the shaft and the hub may decrease.

The present invention has been made in order to solve the above-described problem, and an object thereof is to provide a catheter capable of firmly fixing a shaft and a hub.

### Solution to Problem

A catheter according to the present invention that achieves the above-described object includes: a shaft that is a tubular body in which a lumen extending the shaft from a distal end to a proximal end is formed, and that includes a shaft proximal surface in which the lumen is opened and a shaft outer surface that is an outer peripheral surface of the tubular body; and a hub that is attached to the proximal end of the shaft. The hub includes a tubular accommodation unit that is configured to accommodate the shaft, the accommodation unit includes a hub welded surface that is directly welded to the shaft outer surface, and at least one of the accommodation unit and the hub is formed with a plurality of bubbles at a position close to the hub welded surface.

### Advantageous Effect of Invention

In the catheter configured as described above, the accommodation unit and the hub are welded so as to enter each other by bubbles. Therefore, the shaft and the hub can be firmly fixed.

The accommodation unit may be formed with a plurality of hub bubbles, which are bubbles, at a position close to the hub welded surface. Accordingly, the hub welded surface of the accommodation unit has a complicated shape with irregularities and is welded to the outer surface of the shaft. Therefore, the shaft and the hub can be firmly fixed.

The shaft may be formed with a plurality of shaft bubbles, which are bubbles, at a position close to a shaft welded surface of the shaft outer surface welded to the hub welded surface. Accordingly, the shaft outer surface has a complicated shape with irregularities and is welded to the hub welded surface. Therefore, the shaft and the hub can be firmly fixed.

Both the hub and the shaft may include a plurality of the bubbles, and the hub welded surface and the shaft welded surface of the shaft outer surface welded to the hub welded surface may have irregularities and may be welded so as to enter each other. Accordingly, the shaft and the hub can be fixed more firmly.

### Brief Description of Drawings

Fig. 1 is a plan view illustrating a catheter according to an embodiment.
Fig. 2 is a cross-sectional view illustrating a hub and a proximal portion of a shaft.
Fig. 3 is a cross-sectional view illustrating a distal portion of the hub and the proximal portion of the shaft.
Fig. 4 is a cross-sectional view illustrating the distal portion of the hub before the shaft is fixed.
Fig. 5 is a cross-sectional view illustrating a process of welding the shaft to the hub, in which (A) of Fig. 5 illustrates a state in which welding is started, and (B) of Fig. 5 illustrates a state in which welding is in progress.

### Description of Embodiments

An embodiment according to the present invention will be described hereinafter with reference to the drawings. For convenience of description, dimensional ratios in the drawings may be exaggerated and may be different from actual ratios. In the following description, a side on which a catheter is operated will be referred to as a "proximal side", and a side to be inserted into a living body will be referred to as a "distal side".

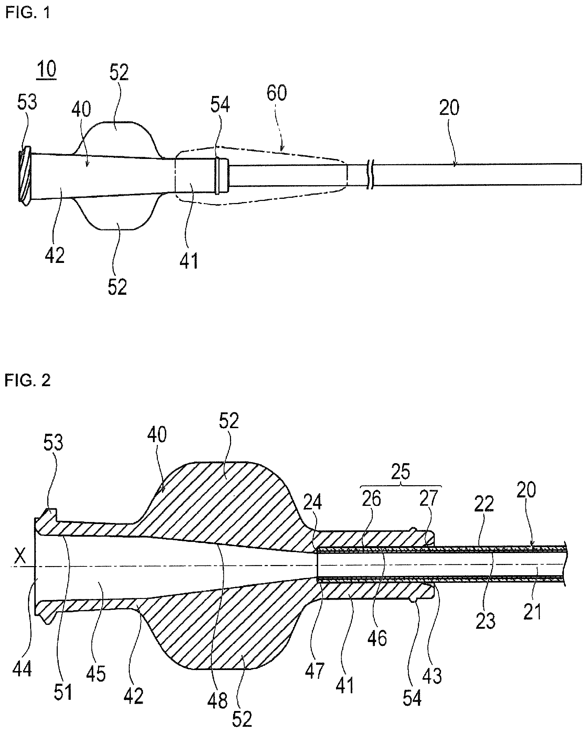

As illustrated in Figs. 1 to 3, a catheter 10 according to the embodiment of the present invention includes a shaft 20 that is an elongated tubular body, a hub 40 fixed to a proximal end of the shaft 20, and a flexible anti-kink protector 60 configured to prevent bending of the shaft 20. In addition to a catheter that supports a guide wire, the catheter 10 may be a guiding catheter, a contrast catheter, or a micro-catheter, or may be a balloon catheter including an inflation lumen or an image diagnosis catheter. In addition, the catheter 10 may be an over-the-wire (OTW) catheter in which a guide wire lumen extending from a distal end of the shaft to the hub is formed, or may be a rapid exchange (RX) catheter in which the guide wire lumen is formed only in a distal portion of the shaft. For example, a guide wire lumen of an RX type balloon catheter is formed from the distal end of the shaft to an opening portion in the middle in an axial center direction of the shaft. An inflation lumen through which a fluid for inflating a balloon of the RX type balloon catheter flows is formed so as to extend from the balloon to a hub at a proximal end of the catheter.

The shaft 20 is formed with a lumen 21 that extends the shaft 20 from a distal end to the proximal end. The shaft 20 includes a shaft outer surface 22, a shaft inner surface 23, and a shaft proximal surface 24.

The shaft outer surface 22 is an outer surface in a radial direction of the shaft 20, which is a tubular body, and extends from the distal end to the proximal end of the shaft 20. The shaft outer surface 22 includes a shaft proximal side outer surface 25 extending from the proximal end toward the distal end of the shaft 20 to a predetermined position. The shaft proximal side outer surface 25 is surrounded by and accommodated in the hub 40. The shaft proximal side outer surface 25 includes a substantially uniform outer diameter along an axial center X of the shaft 20. The shaft proximal side outer surface 25 includes a shaft welded surface 26 welded to the hub 40, and a shaft separated surface 27 that is disposed on the distal side of the shaft welded surface 26 and separated from the hub 40. The shaft separated surface 27 is not welded to the hub 40 and is separated from the hub 40 with a gap therebetween. A plurality of shaft bubbles 32 are formed in the vicinity of the shaft welded surface 26 of the shaft 20. Each of the shaft bubbles 32 includes an inner surface surrounding a closed space.

The shaft inner surface 23 is an inner surface in the radial direction of the shaft 20, which is a tubular body, and extends from the distal end to the proximal end of the shaft 20.

The shaft proximal surface 24 is a surface facing the proximal side at the proximal end of the shaft 20, and is formed by being cut perpendicularly to the axial center X of the shaft 20.

The shaft 20 in the present embodiment includes an inner layer 28 that forms the shaft inner surface 23, an outer layer 29 that forms the shaft outer surface 22, and a reinforcement body 30 that is embedded in the shaft 20.

In addition to a polyamide resin, a polyester resin, a polyolefin resin and a polyurethane resin, examples of a constituent material of the outer layer 29 include a polyamide elastomer, a polyester elastomer, a polyurethane elastomer, a mixture of one or more of these examples, and a mixture of materials having different hardnesses. The outer layer 29 may be formed by arranging materials having different hardnesses so as to become softer from the proximal end toward the distal end.

A constituent material of the inner layer 28 may be the same material as the constituent material of the outer layer 29 described above, or may be a material different from the constituent material of the outer layer 29. The constituent material of the inner layer 28 may be a fluorine-based resin material such as a polytetrafluoroethylene resin in order to improve slidability of an inner peripheral surface of the shaft 20.

The reinforcement body 30 reinforces the shaft 20, and is formed by braiding a plurality of reinforcement wires 31 into a tubular shape. In addition, the reinforcement body 30 may also be formed by spirally winding one or more reinforcement wires 31. The material of the outer layer 29 or the inner layer 28 enters gaps between the plurality of reinforcement wires 31 in the reinforcement body 30. The reinforcement wire 31 is made of a metal such as stainless steel or NiTi.

The hub 40 includes a tubular accommodation unit 41 that is disposed on the distal side and accommodates a proximal portion of the shaft 20, a hub main body 42 that is disposed on a proximal side of the accommodation unit 41, wings 52, a threading projection 53, and an annular projection 54. In the hub 40, a hub lumen 45 is formed which extends from a hub distal end opening 43 formed at a distal end of the accommodation unit 41 to a hub proximal end opening 44 formed at a proximal end of the hub main body 42. The hub lumen 45 includes an accommodation surface 46 that is an inner peripheral surface of the accommodation unit 41, an adjacent surface 47 that faces the shaft proximal surface 24, and a hub passage 48 that is an inner peripheral surface of the hub main body 42.

The accommodation surface 46 includes a hub welded surface 49 directly welded to the shaft welded surface 26 of the shaft proximal side outer surface 25, and a hub separated surface 50 that is separated outward in the radial direction from the shaft separated surface 27 and faces the shaft separated surface 27. The hub welded surface 49 extends from a proximal end of the accommodation surface 46 toward a distal direction. A proximal end of the hub welded surface 49 is connected to the adjacent surface 47. The hub separated surface 50 extends in the distal direction from a distal end of the hub welded surface 49. A gap between the hub separated surface 50 and the shaft proximal side outer surface 25 in the radial direction widens toward the distal direction. It should be noted that the hub separated surface 50 that forms the gap with the shaft proximal side outer surface 25 may not be provided. A plurality of hub bubbles 55 are formed in the vicinity of the hub welded surface 49 of the accommodation unit 41. Each of the hub bubbles 55 includes an inner surface surrounding a closed space.

The adjacent surface 47 is an annular surface facing the distal side, and is formed substantially perpendicular to the axial center X of the shaft 20. An outer side of the adjacent surface 47 in the radial direction is connected to the hub welded surface 49. An inner side of the adjacent surface 47 in the radial direction is connected to a distal end of the hub passage 48.

The hub passage 48 extends from the adjacent surface 47 in a proximal direction. The hub passage 48 is formed in a tapered shape whose inner diameter gradually increases toward the proximal direction. The hub passage 48 is preferably coaxial with the accommodation surface 46 and further coaxial with the lumen 21. An inner diameter of the distal end of the hub passage 48 is preferably substantially equal to an inner diameter of the shaft 20, and is not limited thereto. A part of the tapered hub passage 48 may include a Luer tapered portion 51 connectable to a syringe (not illustrated) . A guide wire or a treatment catheter inserted from the hub proximal end opening 44 smoothly passes through the hub lumen 45 and the lumen 21 and protrudes from a distal end of the catheter 10. Accordingly, the guide wire and the treatment catheter 10 can easily reach a target position such as a lesion area.

The wings 52 are formed so as to protrude from two opposing locations on an outer peripheral surface of the hub main body 42 such that an operator can easily grip and operate the hub 40. The threading projection 53 is formed on the outer peripheral surface of the hub main body 42 on the proximal side. The threading projection 53 can be engaged with a lock type syringe or the like. The annular projection 54 is a projection formed over 360° on an outer peripheral surface of the accommodation unit 41. The annular projection 54 is fittable into a groove formed in an inner peripheral surface of the anti-kink protector 60.

A constituent material of the hub 40 is not particularly limited as long as the material is a thermoplastic resin that can be injection-molded, a material that easily transmits heat or electromagnetic waves is preferable, and specific examples thereof include a polyolefin resin, a polyamide resin, a polycarbonate resin, and a polyester resin.

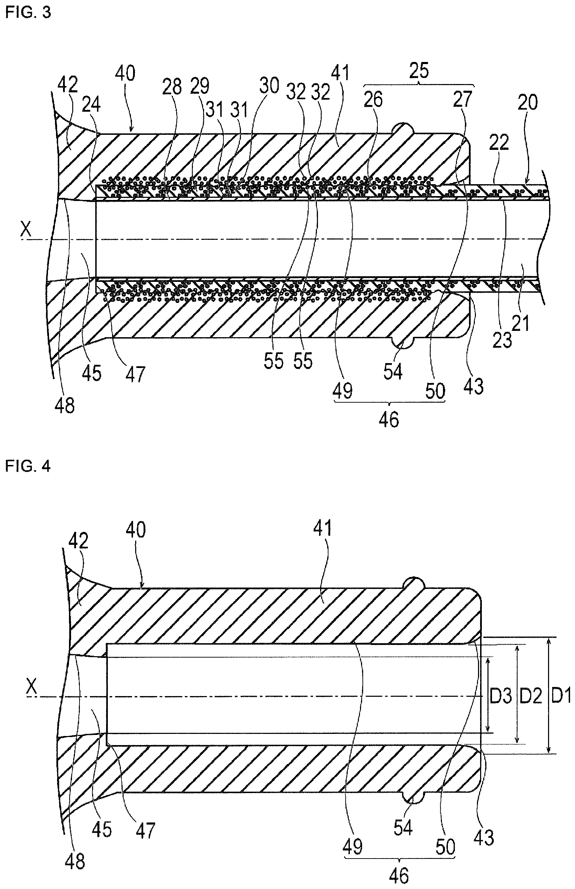

Next, a method of welding the shaft 20 and the hub 40 will be described. As illustrated in Fig. 4, in the hub 40 before the shaft 20 is welded, an inner diameter of the accommodation surface 46 is larger on the distal side than on the proximal side. Specifically, an inner diameter D1 of the hub distal end opening 43 is larger than an inner diameter D2 of the distal end and the proximal end of the hub welded surface 49. In addition, an inner diameter D3 of the distal end of the hub passage 48 is smaller than the inner diameter D2 of the proximal end of the hub welded surface 49. The inner diameter D2 of the hub welded surface 49 is substantially equal to an outer diameter of the shaft proximal side outer surface 25. The inner diameter D1 of the hub distal end opening 43 is, for example, 0.92 mm. The inner diameter D2 of the proximal end of the hub welded surface 49 is, for example, 0.88 mm. The inner diameter D3 of the distal end of the hub passage 48 is, for example, 0.57 mm.

First, the proximal side of the shaft 20 is inserted into the accommodation unit 41, and the shaft proximal surface 24 is abutted against the adjacent surface 47. It should be noted that the shaft proximal surface 24 may not be abutted against the adjacent surface 47 and there may be a gap between the shaft proximal surface 24 and the adjacent surface 47. In addition, a proximal end of the shaft proximal side outer surface 25 is abutted against the proximal end of the hub welded surface 49. It should be noted that the shaft proximal side outer surface 25 may not be abutted against the hub welded surface 49, and there may be a gap between the shaft proximal side outer surface 25 and the hub welded surface 49.

Next, a mandrel (not illustrated) is inserted into the lumen 21 of the shaft 20, and the shaft proximal side outer surface 25 and the accommodation unit 41 of the hub 40 are heated. Accordingly, the shaft proximal side outer surface 25 and the accommodation surface 46 are melted, and the hub welded surface 49 and the shaft welded surface 26 are welded. The hub welded surface 49 and the shaft welded surface 26 may have an integrated structure by being mixed with each other. A heating method is not particularly limited, and examples thereof include a method of emitting electromagnetic waves having a wavelength that allows the electromagnetic waves to be transmitted through the hub 40 and does not allow the electromagnetic waves to be transmitted through the shaft outer surface 22. Since the shaft outer surface 22 does not transmit the electromagnetic waves, the shaft proximal side outer surface 25 is heated and melted at first. Then heat of the shaft proximal side outer surface 25 is transferred to the accommodation unit 41 to melt the accommodation unit 41.

The electromagnetic waves include infrared rays in addition to heat, microwaves, and visible light. The infrared rays are near-infrared rays having a wavelength of about 0.7 µm to 2.5 µm, mid-infrared rays having a wavelength of about 2.5 µm to 4 µm, or far-infrared rays having a wavelength of about 4 µm to 1000 µm, and the infrared rays may be near-infrared rays, mid-infrared rays, far-infrared rays alone or containing two or more types thereof, and may also contain visible light or microwaves.

An electromagnetic wave irradiation method is not particularly limited, and a semiconductor solid-state laser such as a YAG laser using neodymium, a fiber laser, or the like may be used.

The term "electromagnetic waves are transmitted" means that, in addition to being transparent to the naked eye under visible light, a measured transmittance (hereinafter, referred to as the transmittance) is 80% or more, and more preferably 85% or more. The transmittance can be measured by irradiating a sheet having a thickness of 0.4 mm to 0.5 mm prepared by melt-pressing resin pellets with electromagnetic waves having a specific wavelength and using a spectroscopic analyzer, for example, a Fourier transform infrared and near-infrared spectroscopic analyzer. Therefore, since the electromagnetic waves are not limited to visible light, the term "electromagnetic waves are transmitted" includes being transparent with respect to a specific wavelength even if the electromagnetic waves are colored or opaque to the naked eye.

In addition, the term "electromagnetic waves are not transmitted" means that, in addition to being opaque or colored to the naked eye under visible light, the transmittance is less than 80%, preferably less than 10%, and more preferably less than 1%. Therefore, since the electromagnetic waves are not limited to visible light, the term "electromagnetic waves are not transmitted" includes being opaque or absorbed with respect to a specific wavelength even if the electromagnetic waves are transparent to the naked eye.

In the outer layer 29, a pigment that does not transmit or absorbs heat or electromagnetic waves may be mixed in an amount of 0.01 wt% or more and less than 10 wt%, preferably 0.05 wt% or more and 5 wt% or less, and more preferably 0.1 wt% or more and 1 wt% or less with respect to total resin. Alternatively, the outer layer 29 may not contain any pigment, contrast agent, or the like, and the resin forming the outer layer 29 may have a low transmittance with respect to a specific wavelength. Alternatively, in the outer layer 29, a metal having X-ray contrast properties may be mixed in place of, or together with the pigment.

The pigment is not particularly limited as long as the pigment is a pigment that develops white, black, blue, red, or yellow or a mixture thereof, and a black pigment, for example, carbon black is preferable as a pigment that easily absorbs electromagnetic waves. The X-ray contrast agent is, for example, a compound of gold, bismuth, and tungsten, and is more preferably in the form of powder.

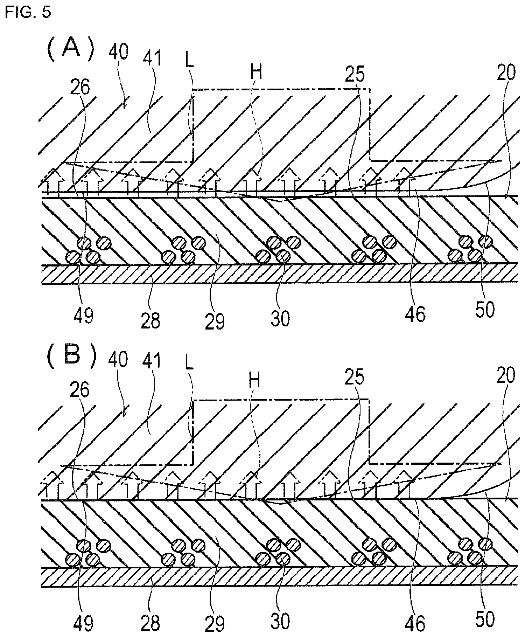

For example, as illustrated in (A) of Fig. 5, when the shaft 20 and the hub 40 are welded to each other by irradiation with an infrared laser L, electromagnetic waves transmitted through the hub 40, which is transparent to a wavelength of the irradiated infrared laser L, are absorbed by the opaque resin, pigment, or the like of the outer layer 29 of the shaft 20 and mainly generate heat. Accordingly, the resin of the outer layer 29 melts and transfers heat H to the accommodation unit 41 of the hub 40, and at least a part of the accommodation surface 46 melts. As illustrated in (B) of Fig. 5, a diameter of the melted accommodation surface 46 decreases, and the accommodation surface 46 is brought into close contact with the shaft proximal side outer surface 25, and is welded to the shaft proximal side outer surface 25. Accordingly, the hub welded surface 49 and the shaft welded surface 26 are welded and formed. At this time, the accommodation surface 46 melts and the diameter thereof decreases, and the outer peripheral surface of the accommodation unit 41 hardly melts and hardly deforms since heat is hardly transmitted. Therefore, due to the diameter decrease of the accommodation surface 46, a material constituting the accommodation surface 46 flows so as to fill a gap between the accommodation surface 46 and the shaft proximal side outer surface 25, and as a result, a length of the accommodation unit 41 along the axial center X is reduced. Accordingly, the accommodation unit 41 and the shaft proximal side outer surface 25 are favorably welded without any gap therebetween. In addition, the shaft proximal surface 24 and the adjacent surface 47 are also melted and welded by the heat generation of the outer layer 29. It should be noted that the shaft proximal surface 24 may not be welded to the adjacent surface 47.

When the shaft proximal side outer surface 25 and the accommodation surface 46 are welded, as illustrated in Fig. 3, the shaft bubbles 32 are formed in the vicinity of the shaft welded surface 26 of the shaft 20, and the hub bubbles 55 are formed in the vicinity of the hub welded surface 49 of the accommodation unit 41. The bubbles are generated by evaporation of materials, mixing of air, or the like. By forming the shaft bubbles 32 and the hub bubbles 55, the shaft welded surface 26 and the hub welded surface 49 have irregularities and are mixed with each other in a complicated manner, and are formed to enter each other. Therefore, the hub 40 and the shaft 20 are fixed to each other with a high coupling force. The hub welded surface 49 and the shaft welded surface 26 may have an integrated structure by being mixed with each other. It should be noted that only one of the shaft bubbles 32 and the hub bubbles 55 may be formed. In this case, the hub welded surface 49 and the shaft welded surface 26 are still mixed with each other in a complicated manner, and are still formed to enter each other. Therefore, the hub 40 and the shaft 20 are fixed to each other with a high coupling force.

The hub separated surface 50 located at the distal end of the hub welded surface 49 is not welded to the shaft separated surface 27, and a gap is maintained between the hub separated surface 50 and the shaft separated surface 27. When the entire hub welded surface 49 located on the proximal side of the hub separated surface 50 is welded to the shaft welded surface 26, the irradiation of the infrared laser L is stopped. Accordingly, fixation of the hub 40 and the shaft 20 is completed.

As described above, the catheter 10 according to the present embodiment is the catheter 10 including: the shaft 20 that is the tubular body in which the lumen 21 extending the shaft 20 from the distal end to the proximal end is formed, and that includes the shaft proximal surface 24 in which the lumen 21 is opened and the shaft outer surface 22 that is the outer peripheral surface of the tubular body; and the hub 40 attached to the proximal end of the shaft 20. The hub 40 includes the tubular accommodation unit 41 configured to accommodate the shaft 20, the accommodation unit 41 includes the hub welded surface 49 directly welded to the shaft outer surface 22, and at least one of the accommodation unit 41 and the hub 40 is formed with a plurality of bubbles at a position close to the hub welded surface 49.

In the catheter 10 configured as described above, the accommodation unit 41 and the hub 40 are welded so as to enter each other by the plurality of bubbles. Therefore, the shaft 20 and the hub 40 can be firmly fixed. Therefore, the shaft 20 can be prevented from being detached from the hub 40 in a case where a high pressure of a contrast agent injected into the catheter 10 is applied, a case where a tensile force acts between the hub 40 and the shaft 20 when the shaft 20 is pulled out from a body, or the like.

In addition, the accommodation unit 41 is formed with the plurality of hub bubbles 55, which are bubbles, at a position close to the hub welded surface 49. Accordingly, the hub welded surface 49 of the accommodation unit 41 has a complicated shape with irregularities and is welded to the shaft outer surface 22. Therefore, the shaft 20 and the hub 40 can be firmly fixed.

In addition, the shaft 20 is formed with the plurality of shaft bubbles 32, which are bubbles, at a position close to the shaft welded surface 26 of the shaft outer surface 22 welded to the hub welded surface 49. Accordingly, the shaft outer surface 22 has a complicated shape with irregularities and is welded to the hub welded surface 49. Therefore, the shaft 20 and the hub 40 can be firmly fixed.

In addition, both the hub 40 and the shaft 20 include the plurality of bubbles, and the hub welded surface 49 and the shaft welded surface 26 of the shaft outer surface 22 welded to the hub welded surface 49 have irregularities and are welded so as to enter each other. Accordingly, the shaft 20 and the hub 40 can be fixed more firmly.

The present invention is not limited to the above-described embodiment, and various modifications can be made by those skilled in the art within the technical idea of the present invention. For example, the shaft 20 may be heated by high-frequency induction heating during which heating is performed by electromagnetic induction. An electromagnetically-induced conductor is, for example, the reinforcement body 30.

### Reference Signs List

- 10: catheter
- 20: shaft
- 21: lumen
- 22: shaft outer surface
- 23: shaft inner surface
- 24: shaft proximal surface
- 25: shaft proximal side outer surface
- 26: shaft welded surface
- 27: shaft separated surface
- 28: inner layer
- 29: outer layer
- 30: reinforcement body
- 32: shaft bubble (bubble)
- 40: hub
- 41: accommodation unit
- 42: hub main body
- 43: hub distal end opening
- 44: hub proximal end opening
- 45: hub lumen
- 46: accommodation surface
- 47: adjacent surface
- 48: hub passage
- 49: hub welded surface
- 50: hub separated surface
- 55: hub bubble (bubble)
- 60: anti-kink protector

## Claims

1. A catheter (10) comprising:
a shaft (20) that is a tubular body in which a lumen (21) extending in the shaft (20) from a distal end to a proximal end is formed, and that includes a shaft proximal surface (24) in which the lumen (21) is opened and a shaft outer surface (22) that is an outer peripheral surface of the tubular body; and
a hub (40) attached to the proximal end of the shaft (20), wherein
the hub (40) includes a tubular accommodation unit (41) configured to accommodate the shaft (20),
the accommodation unit (41) includes a hub welded surface (49) directly welded to the shaft outer surface (22), **characterized in that**
at least one of the accommodation unit (41) and the hub (40) is formed with a plurality of bubbles at a position close to the hub welded surface (49).

2. The catheter (10) according to claim 1, wherein
the accommodation unit (41) is formed with a plurality of hub bubbles (55), which are bubbles, at a position close to the hub welded surface (49).

3. The catheter (10) according to claim 1 or 2, wherein
the shaft (20) is formed with a plurality of shaft bubbles (32), which are bubbles, at a position close to a shaft welded surface (26) of the shaft outer surface (22) welded to the hub welded surface (49).

4. The catheter (10) according to claim 1, wherein
both the hub (40) and the shaft (20) include a plurality of the bubbles, and the hub welded surface (49) and a shaft welded surface (26) of the shaft outer surface (22) welded to the hub welded surface (49) have irregularities and are welded so as to enter each other.

## Patentansprüche

1. Katheter (10), umfassend:
einen Schaft (20), der ein röhrenförmiger Körper ist, in dem ein Lumen (21) ausgebildet ist, das sich in dem Schaft (20) von einem distalen Ende zu einem proximalen Ende erstreckt, und der eine proximale Oberfläche (24) des Schafts, in der sich das Lumen (21) öffnet, und eine äußere Oberfläche (22) des Schafts, die eine äußere Umfangsfläche des röhrenförmigen Körpers ist, aufweist; und
eine Nabe (40), die an dem proximalen Ende des Schafts (20) angebracht ist, wobei
die Nabe (40) eine röhrenförmige Aufnahmeeinheit (41) umfasst, die so konfiguriert ist, dass sie den Schaft (20) aufnimmt,
die Aufnahmeeinheit (41) eine Nabenschweißfläche (49) aufweist, die direkt mit der Schaftaußenfläche (22) verschweißt ist, **dadurch gekennzeichnet, dass**
mindestens eine von der Aufnahmeeinheit (41) und der Nabe (40) mit einer Vielzahl von Blasen an einer Position nahe der Nabenschweißfläche (49) ausgebildet ist.

2. Katheter (10) nach Anspruch 1, wobei
die Aufnahmeeinheit (41) mit einer Vielzahl von Nabenblasen (55), welche Blasen an einer Position nahe der Nabenschweißfläche (49) sind, ausgebildet ist.

3. Katheter (10) nach Anspruch 1 oder 2, wobei
der Schaft (20) mit einer Vielzahl von Schaftblasen (32) ausgebildet ist, welche Blasen an einer Position nahe der Schaftschweißfläche (26) der äußeren Oberfläche (22) des Schaftes sind, die an die Nabenschweißfläche (49) geschweißt ist.

4. Katheter (10) nach Anspruch 1, wobei
sowohl die Nabe (40) als auch der Schaft (20) eine Vielzahl von Blasen aufweisen und die Nabenschweißfläche (49) und eine Schaftschweißfläche (26) der äußeren Oberfläche (22) des Schaftes, die mit der Nabenschweißfläche (49) verschweißt ist, Unregelmäßigkeiten aufweisen und so verschweißt sind, dass sie ineinander übergehen.

## Revendications

1. Cathéter (10) comprenant :
une tige (20) qui est un corps tubulaire dans lequel est formée une lumière (21) s'étendant dans la tige (20) d'une extrémité distale à une extrémité proximale, et qui comporte une surface proximale (24) de tige dans laquelle la lumière (21) est ouverte et une surface externe (22) de tige qui est une surface périphérique externe du corps tubulaire ; et
une embase (40) fixée à l'extrémité proximale de la tige (20), dans lequel
l'embase (40) comporte une unité de réception tubulaire (41) configurée pour recevoir la tige (20),
l'unité de réception (41) comporte une surface soudée (49) d'embase directement soudée à la surface externe (22) de tige, **caractérisé en ce que**
au moins l'une de l'unité de réception (41) et de l'embase (40) est formée d'une pluralité de bulles en une position proche de la surface soudée (49) d'embase.

2. Cathéter (10) selon la revendication 1, dans lequel l'unité de réception (41) est formée d'une pluralité de bulles (55) d'embase, qui sont des bulles, en une position proche de la surface soudée (49) d'embase.

3. Cathéter (10) selon la revendication 1 ou 2, dans lequel la tige (20) est formée d'une pluralité de bulles (32) de tige, qui sont des bulles, en une position proche d'une surface soudée (26) de tige de la surface externe (22) de tige soudée à la surface soudée (49) d'embase.

4. Cathéter (10) selon la revendication 1, dans lequel l'embase (40) et la tige (20) comportent toutes deux une pluralité des bulles, et la surface soudée (49) d'embase et une surface soudée (26) de tige de la surface externe (22) de tige soudée à la surface soudée (49) d'embase présentent des irrégularités et sont soudées de manière à pénétrer l'une dans l'autre.
